Europäisches Patentamt

European Patent Office

Office européen des brevets

(19)

(11) Publication number: **0 372 892**
**A2**

# (12) EUROPEAN PATENT APPLICATION

(21) Application number: **89312636.7**

(22) Date of filing: **04.12.89**

(51) Int. Cl.5: **A61M 5/31**

(30) Priority: **02.12.88 PT 89148**

(43) Date of publication of application:
**13.06.90 Bulletin 90/24**

(84) Designated Contracting States:
**BE CH DE ES FR GB IT LI SE**

(71) Applicant: **Roseiro, Antonio Henriques**
**Rue Cabo da Bos Esperanca No. 6-C, Cova**
**da Piednde**
**Almada(PT)**

(72) Inventor: **Roseiro, Antonio Henriques**
**Rue Cabo da Bos Esperanca No. 6-C, Cova**
**da Piednde**
**Almada(PT)**

(74) Representative: **Alexander, Thomas Bruce et**
**al**
**Boult, Wade & Tennant 27 Furnival Street**
**London EC4A 1PQ(GB)**

(54) **Plastic material syringe for dental treatment.**

(57) A plastic material syringe for use in dental medicine is disclosed. The shape of this syringe has been ergonomically studied and adapted to working inside the mouth. The ergonomic shape of the body and piston cuff allows for a great firmness and control over work execution. The invention could allow for the utilization of the conventional ampoules and needles. The syringe is made of plastic material has for its basic goal the possibility of disposability. Its low production cost warrants this possibility. Furthering the sanitary interest, its use results in economic rewards if the reduction of sterilization operations is taken in account as well as the greater flexibility resulting from having always available syringes. One embodiment of this syringe has the additional advantage of having been designed specifically to work with children, thus having a device for obscuring the needle and a colourful adornment able to give it an inoffensive aspect.

FIG. 1.

## PLASTIC MATERIAL SYRINGE FOR DENTAL TREATMENT

The invention relates to a syringe intended for dental medicine.

The syringes currently in use as an integral part of dental medicine equipment raise some problems whose resolution seems urgent, and to which the presently offered model gives an answer.

Endowed with special characteristics such as the insertion mechanisms for the ampoules and needles and also the way in which they adapt themselves to the pratictioner hand, the syringes used nowadays in dental medicine are multiple utilization metallic instruments needing a very effective sterilization each time they are used.

In fact, the space inside the dental ambulatory constitutes a high risk zone in which contamination is a concern, due to the kind of work that it carried on there, thus being of utmost relevance to what World Health Organization suggests for patient's protection.

For a long time the problem of an effective sterilization of dental equipment has been part of a more or less trivial everyday routine where the true supervisor has been the user's professional deontology, and that of his co-workers. With the development of AIDS problems and the responsibility associated with it the security of sterilization has changed to become a truly dramatic question in which the patient is already by himself one of the more concerned critics of its rigorous execution.

Thus a solution imposes itself that can obviously warrant an high degree of security and effectiveness without taxing significantly the already onerous process associated to this kind of treatment, and allowing as much as possible a greater agility for the response the ambulatories can give.

An additional risk factor which can not be underestimated is due to the aggressive aspect that traditional syringes assume, particularly as far as the treatment of children is concerned. In fact, it remains a concern in this domain to obtain from the patient a positive attitude, which is regarded as very difficult when the syringe's needle is exhibited before these patients' eyes. With the now presented model an effective solution is offered to the problem.

Finally, better ergonomics are commended, namely in the adaptation of the syringe to the hand of the user, and in the foreseen use by left-handed people.

The present invention provides a syringe for use in dental medicine made of plastic material, and to be used only once. Its sterilization is carried out by the manufacturer and it is verified by the hermetically sealed package in which it is sold.

According to the present invention there is provided a plastic material syringe for dental treatments, the plastic material being suitable for purposes of sterilization, said syringe consisting of a cylindrical body and a piston with a cuff, wherein one of the bases of said cylindrical body has a hole with an external protruding screw thread for positioning of the needles, and the other base is open, and annexed to this last one is a screw thread on the outside of the cylindrical body, and having further a piston comprising a cursor with transversal marks associate to a cuff, said cursor crossing through a cylindrical part with an interior screw able to co-operate with the screw thread installed near the open base of said cylindrical body.

Following the model of "disposable" syringes, this one is characterised by being specifically adapted to dental medicine operations, namely through:

- Ergonomically moulded body and piston cuff in order to allow for an effective adaptation to the hand of the user. An embodiment for left-handed people shall be obtainable.
- Closed central body for the installation of standard ampoules currently in use with metallic syringes. The dosing of the fluid injected is verified through the marks existing upon the piston cursor.
- Needle replacement conventional device.
- Great endurance.

Among the problems associated with conventional syringes it is important to point out its somewhat aggressive aspect especially when the patient is a child. That aggressiveness comes primarily from the fact that the exhibition of a needle and the certain "look" of a chirugical instrument do not mask a painful intention. From this to the negative expectancy and corresponding reactions in the patient there is just a little step to cross. One of the embodiments of the inventive syringe is designed with to all the above characteristics in, and provides a successful attempt for easing the image of these type of instruments.

This effect is reached through:
- introduction of a device that shades the needle giving to the above-mentioned syringe a harmless appearance. The needle is exposed only in the moment and place where it is to be used.
-adornment with a colourful infantile pattern, turning it into something visually agreeable, and thus contributing to inhibit the appearance of rejection attitudes.

Preferred embodiments of the present invention will now be described with reference to the accompanying drawings, in which:

Fig. 1 shows one embodiment of the syringe in an unassembled state.

Fig. 2 shows the same embodiment of the syringe as fig. 1, in an assembled state.

Fig. 3 shows a second embodiment of the syringe in an unassembled state.

Fig. 4 shows the second embodiment of the syringe shown in fig. 3, in an assembled state.

Fig. 5 shows a third embodiment of the syringe comprising additionally a sheath.

Fig. 6 shows the embodiment of the syringe shown in fig. 5 when assembled.

Fig. 7 shows a fourth embodiment of the syringe with the prehensile member comprising protrusions shaped in the form of a human face.

Fig. 8 shows the embodiment of fig. 7 when assembled.

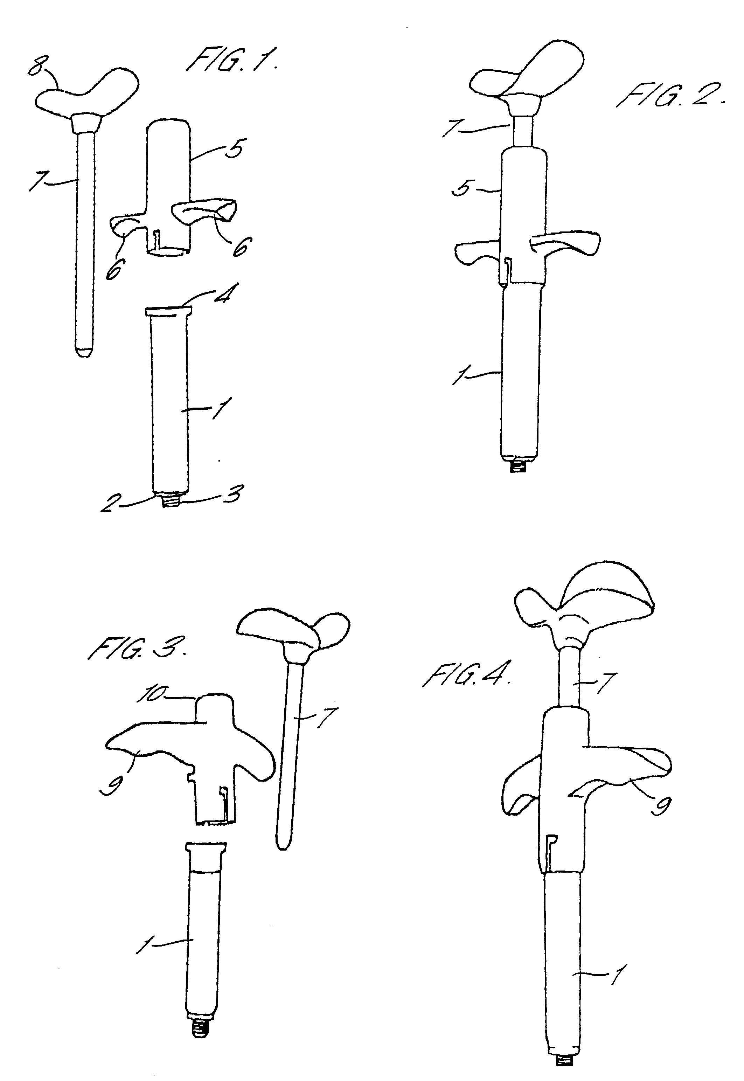

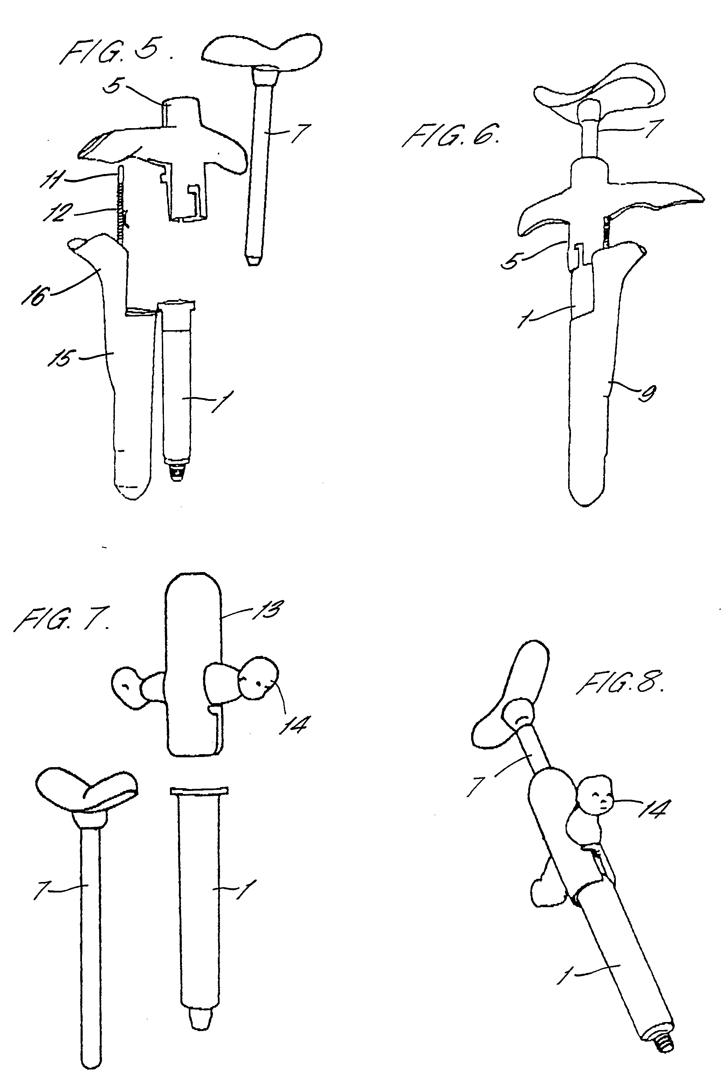

In one embodiment (shown in fig. 1) the present invention comprises a cylindrical body (1), a piston (7) and a prehensile member (5). The cylindrical body has one open end and one closed end. At the closed end (2) of the cylindrical body (1) there is disposed an external protrusion (3). The protrusion has an external screw thread and is hence designed to enable a needle to be attached to the syringe. A passage runs through the protrusion, in the longitudinal direction, that is between the two flat faces of the cylinder, so that the interior of the syringe is connected to the needle. The open end (4) of the cylindrical body (1), when the syringe is in use, is connected to a prehensile member (5), by such means as a screw thread on the outside of the open end or by the positive interaction of protrusions on the outside of the cylinder with slots in the prehensile member. The prehensile member (5), or "cuff", is of a generally cylindrical shape and comprises two protrusions (6) perpendicular to the longitudinal axis (axis of symmetry) of the cylindrical portion of prehensile member. The prehensile member is hollow and its interior diameter is the same as or greater than the exterior diameter of the piston(7). Therefore, in use, (as shown in fig. 2) the piston may slide within the prehensile member. The arrangement enables injections to be performed within the mouth of the patient. Since the invention is made of plastics it is cheap to produce and may be thrown away after use. In use, pressure is applied to the top of the piston (8) which slides down within the prehensile member to cause the contents of the cylindrical body to be expelled through the passage at the closed end thereof.

In a further embodiment of the present invention (fig. 3) the syringe comprises a specially shaped prehensile member (10). The prehensile member (10) has special ergonomically shaped protrusions (9) which are designed to aid the user of the syringe.

In a further embodiment (fig. 5) a sheath (15) is used with the syringe. The sheath is of a generally hollow cylindrical shpae. One end of the sheath has a frusto-conical portion. There exists a hole in the frusto-conical portion of a diameter sufficient to accomodate a needle. The other end of the sheath is open so that the sheath may be positioned covering the syringe. A shaped protrusion (16) at one side of the sheath serves to enable the sheath (9) to be attached to the prehensile member (5) and also to cover the means by which one is joined to the other. The sheath (9) is attached to the prehensile member (5) by a hook (11). The sheath (15) is attached to the spring (12) at the end of which there is attached a hook (11). The spring (11) acts to keep the sheath (9) in contact with the cylindrical body (1). The spring may take the form of a conventional metal spring or a strip of resilient material. The sheath serves to obscure from the view of the patient. The sheath (15), by hiding the needle from view, adds to ease the state of mind of the patient.

In another embodiment of the present invention (fig. 7), designed for the younger patients, the prehensile member (13) comprises specially designed protrusions (14). These protrusions take the form of human faces. The syringe of the embodiment is also brightly coloured. The embodiment is designed to allay the fears of younger patients and may incorporate a brightly coloured sheath to obscure the needle from view.

## Claims

1. A plastic material syringe, said plastic material being of the sterilizable type, said syringe consisting of a cylindrical body and a piston with a cuff, wherein one of the bases of said cylindrical body has a hole with an external protruding screw thread for positioning of the needles, and the other base is open, and annexed to this last one is a screw thread outside the cylindrical body, and having further a piston comprising a cursor with transversal marks associate to a cuff, said cursor crossing through a cylindrical part with an interior screw able to cooperate with the screw thread installed near the open base of said cylindrical body.

2. A plastic material syringe for dental treatments as claimed in Claim 1, wherein there is near the open base of said cylindrical body a prehensile bar comprising two simple opposed protrusions adequate for fingers settlement, and the cursor-cuff assembly has a "T" shape in order to allow for a precise placing of the thumb's root.

3. A plastic material syringe for dental treatment as claimed in Claim 1, wherein there is near said open base of said cylindrical body a prehension bar comprising two opposed protrusions anatomically moulded in order to permit a firm prehension by means of three fingers of the right hand, and with said cursor cuff of said piston being anatomically moulded in order to allow for the placing in extension of the thumb's root and adjacent surface right hand's palm.

4. A plastic material syringe for dental treatment as claimed in Claim 3, wherein there is an anatomical moulding for the left hand.

5. Plastic material syringe for dental treatment as claimed in any one of the preceding Claims, wherein the cylindrical body is adourned with bright colours and infantile patterns.

6. Plastic material syringe for dental treatment as claimed in any one of the preceding Claims wherein there is a device to shade the needle being said device shaped as an hollow cylinder with an interior diameter able to permit its sliding along the cylindrical body of said syringe, said hollow cylinder ending in one of its extremities by a perforated truncated cone, and being open in the other end, and having near said open end a slight protrusion, and a spring able to maintain said hollow cylinder in its shading needle position and a hook able to maintain retracted near said main body prehension bar said hollow cylinder.

7. A syringe adapted for use in dental medicine, characterised in that the syringe is made of plastics and is intended for one use only.

8. A syringe comprising a cylindrical body, a piston and a prehensile member, characterised in that the syringe further comprises a sheath adapted to surround said cylindrical body and obscure from sight a needle attached to the cylindrical body, said sheath comprising means to attach it to the prehensile member, said syringe being formed of plastics.

9. A syringe as claimed in Claim 7 or Claim 8 wherein the prehensile member comprises two protrusions which are moulded in the shape of figures, human, animal or otherwise.

10. A syringe comprising a cylindrical body, a piston and a prehensile member, said cylindrical body having disposed of the closed end thereof a cylindrical protrusion with an exterior screw thread, said cylindrical protrusion containing a passage therethrough, said passage having one opening in the interior of the said cylindrical body and one other opening to the exterior of the said cylindrical body at the end of the said protrusion, said cylindrical body also comprising an open end and means existing on the exterior of the open end to enable the cylindrical body to be attached to the prehensile member, said prehensile member being of a hollow cylindrical shape with two protrusions perpendicular to the longitudinal axis of the member, the interior diameter of the prehensile member corresponding to the diameter of the piston, characterised in that the syringe is made of plastics and is intended for one use only.

FIG.1.

FIG.2.

FIG.3.

FIG.4.

FIG. 5.

5

11

12

16

15

1

7

FIG. 6.

7

5

1

9

FIG. 7.

13

14

7

1

FIG. 8.

7

14

1